# EUROPEAN PATENT SPECIFICATION

(11) **EP 3 981 455 B1**
(45) Date of publication and mention of the grant of the patent: **17.01.2024**
(21) Application number: 21174890.0
(22) Date of filing: 20.05.2021
(51) Int. Cl.: A61M 16/06, A62B 18/08, A41D 13/11

(54) **A RESPIRATORY MASK COMPRISING A HEADGEAR**
ATEMMASKE MIT KOPFBEDECKUNG
MASQUE RESPIRATOIRE COMPRENANT UN CASQUE

(30) Priority: 08.10.2020 EP 20200679
(43) Date of publication of application: 13.04.2022
(73) Proprietor: Air Liquide Medical Systems, 92160 Antony (FR)
(72) Inventor: MASSARO, Paolo, 25073 Bovezzo (IT); LEBRET, Marius, 92160 Antony (FR); VERGNES, Marie, 92160 Antony (FR); TESNIERE, Eva, 92160 Antony (FR)
(74) Representative: Air Liquide

(56) References cited:
- EP-A1- 1 902 743
- EP-A1- 3 082 581
- EP-A1- 3 369 451
- WO-A1-2010/135785
- WO-A1-2012/045127
- WO-A1-2014/025267
- US-A- 3 457 564
- US-A- 4 607 398
- US-A1- 2012 199 131

## Description

The invention concerns an improved headgear structure for a respiratory mask and a respiratory mask equipped with such a headgear structure, such as a nasal or facial mask covering the nose or the nose and mouth of a patient, in particular for adult or pediatric uses in the treatment of respiratory conditions or diseases affecting adults, children, toddlers, infants or newborns.

Nasal or facial respiratory masks are commonly used for delivering non-invasive positive pressure ventilation (NPPV) or for continuous positive airway pressure (N-CPAP) therapy in disordered breathing conditions, such as obstructive sleep apnea (OSA) or chronic obstructive pulmonary disease (COPD). In both cases, the mask delivers a flow of breathable gas for, or to assist in, patient respiration. Examples of masks are given by EP-A-462701, EP-A-462701, EP-A-874667, EP-A-1972357 and WO-A-00/57942.

Such masks typically comprise a hollow shell, usually made of polymer or silicone, defining a breathing chamber that receives at least a part of the patient's nose in the case of a nasal mask, or both the nose and the mouth of the patient in the case of a facial mask. The hollow shell or body receives the gas from a gas supply line, fixed to an inlet orifice arranged in the mask body by means of a hollow gas connector, for delivering the respiratory gas, such as air under pressure, into the breathing chamber of the shell. A soft face-contacting cushion comes into contact with the patient's face and conforms to the various facial contours of the patient face thereby ensuring gas tightness. The cushion is usually made of soft, resilient elastomeric material, such as silicone.

A respiratory mask also includes a headgear for correctly positioning, maintaining and/or securing the mask on the head of a patient. The headgear is fixed directly to the mask body and/or to an upwardly projecting arm that is itself either directly fixed to the mask body, or to a supplementary front piece, usually called "shroud" or "retainer", that is arranged in front of the mask body, as disclosed by WO-A-0074758, EP-A-1057494 or EP-A-2708258.

Typically, a headgear comprises straps, such as woven straps or the like, that form a kind of flexible helmet that is arranged on the head of the patient.

When the mask is positioned on the face of a user, such as a patient, the straps of the headgear have to be tightened up so that the mask fits the facial morphology of the user, and is maintained in said desired position. This tightening up action may lead to an excessive crushing of the cushion on the nasal bridge region, when the straps of the headgear of the mask are overly tightened. This may lead to gas leaks and discomfort for the patient. Obviously, insufficient tightening can also lead to similar issues, i.e. gas leaks and discomfort for the patient. In both cases, the mask is less efficient for delivering a gas therapy, which is not acceptable.

An over- or an under- tightening generally results from a wrong positioning of the straps of the headgear so that the tension exerted by the straps on the mask is excessive or, conversely, insufficient, knowing that it is generally not easy for a patient to correctly set up the mask and the headgear on his/her head.

US-A-3,457,564 proposes a mask comprising a headgear with straps comprising transverse ribs and dimples carried by the straps, which cooperates with buckles for adjusting the headgear on the patient's head. The ribs are arranged in rows on the straps. Each rib corresponds to a given adjustment setting of the headgear. However, with such a mask, it is not always easy for a user to figure out how to correctly adjust the strap of the headgear as nothing helps him/her to know which ribs should be selected to obtain a suitable tightening.

EP-A-1902743 teaches a headgear for a respiratory mask comprising indicating means responsive to an adjustment parameter of the headgear for indicating attainment of a desired adjustment setting of the headgear. The indicating means comprises a set of indicia that arranged to change in elongation responsive to the headgear tension. Those indicia comprise ellipses of different shapes that may be optionally labelled with tension settings, e.g., 1, 2, 3, 4..., or with pressure values. When the headgear strap is placed under tension, the headgear material stretches and the ellipses become more and more circular, depending on the tension applied. The most circular ellipse indicates the tension setting. However, it can be difficult for some patients to correctly adjust the headgear based on the shapes of the set of ellipses.

The following documents are also disclosing different masks and corresponding headgears, namely EP 3,082,571 B1, US 2012/0199131 A1, WO 2012/045127, WO 2010/135785, WO 2014/025267 and EP1,902,743 A1.

Generally speaking, with known masks and headgears, the risks of gas leaks and discomfort for the patient, due to a wrong adjustment, can be significant.

In this context, the problem to be solved is to provide an improved headgear, and a mask equipped with such an improved headgear, that helps the patient to always apply an equal tightening to the straps of the headgear thereby allowing he/her to correctly set up the mask and the headgear on his/her head, and thus avoiding or limiting the risks of leaks and discomfort resulting from an over- or an under-tightening of the straps.

The invention is defined in independent claim 1. Preferred embodiments are disclosed in the dependent claims. The solution of the present disclosure concerns a headgear for a respiratory mask comprising several flexible straps, wherein at least one of said flexible straps :
- comprises an outer face located externally, a free end with a tip and detachable securing means (i.e. detachable fixation means) arranged on said outer face,
- is configured for being foldable so that, when folded, the free end of said flexible strap can be positioned by a user in several different positions located along the outer face of said at least one flexible strap, and removably secured in one of said several different positions by means of the detachable securing means, and
- further comprises several markings arranged on the outer face of said at least one flexible strap, said several markings corresponding to different tightenings (i.e. tightening-up settings) of said at least one flexible strap,
wherein, when said at least one flexible strap is folded, the tip of the free end of said at least one flexible strap is positioned vis-à-vis one given marking of said several markings so as to designate said given marking thereby assisting the user in choosing a desired tightening (i.e. adjustment) of said at least one flexible strap.

The headgear for a respiratory mask according to the present disclosure can further comprise one or more of the following additional features:
- the (each) free end is terminated by a tip, i.e. a terminal extremity.
- each flexible strap comprises an elongated main body (i.e. ribbon or band shape) terminated by a free end, preferably a free end comprising a tip.
- at least one flexible strap comprises an elongated main body comprising a core region terminated by the free end, preferably at least two flexible straps.
- at least two flexible straps comprise an elongated main body terminated by a free end with a tip, preferably four straps.
- the elongated main body comprises the core region having a first width (W1) and the free end having a second width (W2), wherein the first width (W1) is greater than the second width (W2), i.e. W1 > W2.
- each marking represents or corresponds to a given position of said several different positions.
- when folded, the free end of the flexible strap(s) is maintained along the outer face of said flexible strap(s), in the desired position, by means of the detachable securing means.
- when folded, the free end of the flexible strap(s) is maintained in contact with the outer face of said flexible strap(s) by means of the detachable securing means.
- when folded, the free end of the flexible strap(s) is maintained in contact with the outer face of said flexible strap(s) thereby forming a fixation loop.
- when folded, the free end of the flexible strap(s) comes into contact with the outer face of the core region of the elongated main body of said flexible strap(s) thereby forming a fixation loop.
- when folded, the free end of the flexible strap(s) is attached to the outer face of a core region of the elongated main body of said flexible strap(s).
- when folded, the free end of one or several flexible straps is maintained along and in contact with the outer face of said one or several flexible straps by means of the detachable securing means, in a desired position wherein the tip of the free end of said one or several flexible straps designates (i.e. is facing) one given marking of the several markings, i.e. said given marking represents or corresponds to said desired position.
- it comprises at least two flexible straps comprising detachable securing means and markings, preferably at least four flexible straps comprising detachable securing means and markings.
- each flexible strap forms, when folded, a fixation loop.
- the little-hook region arranged on the elongated main body are configured for being attached to the little-loop region arranged on the free end of at least one flexible strap, when said free end is folded over the core region of said elongated main body, thereby forming a fixation loop.
- each flexible strap forms, when folded, a fixation loop and has its free end in (quasi)contact with its outer face, preferably in its core region.
- one or several flexible straps comprise a free end with a tip having a flat rounded-shape, such as a flat half-disk shape or similar.
- alternatively, one or several flexible straps comprise a free end with a tip having a flat angular-shape, such as a triangular or a trapezoidal flat shape, or similar.
- at least two flexible straps comprise a free end with a colorized tip, i.e. comprising a colored layer or structure, preferably a colored layer or structure (at least partially) made of polymer material.
- the colorized tip can be white, yellow, orange, red, blue, green or any other colours, i.e. made of a colorized polymer material.
- the colored layer or structure is carried by a fabric material forming the/each strap(s), namely fabric-material having a band- or ribbon-shape.
- the tip(s) is made of polymer material.
- the tip(s) can be formed by thermo-printing, thermo-welding, pad-printing, RF-welding, ultrasonic welding or the like.
- the tip(s) is made of a polymer material that is secured to a fabric material forming the strap(s).
- each flexible strap comprises an inner surface inwardly-oriented so as to be in contact with the patient's head, when the headgear is worn by the patient, i.e. in use.
- the outer surface is outwardly-oriented, i.e. located opposite to the inner surface.
- the flexible straps are foldable.
- the flexible straps are made of a flexible material so as to be foldable, i.e. bentable.
- the flexible straps are made of a fabric, such as UBL and/or Lycra^{®}, generally laminated with a polyurethane foam as filling material, or similar materials.
- the flexible straps are flat.
- the flexible straps have a ribbon- or band-shape, or the like.
- the detachable securing means comprise a little-loop region comprising a multitude of little loops and a little-hook region comprising a multitude of little hooks cooperating together for ensuring a desired removable attachment of the little-hook region to the little-loop region.
- the detachable securing means comprise a Velcro^{®}-type attachment system.
- when folded, the free end of the flexible strap(s) is detachably secured to the outer face of the core region of the elongated main body of said flexible strap(s) by means of the detachable securing means.
- the free end of the flexible strap(s) comprises the little-hook region and the core region of the elongated main body of the flexible strap(s) comprises the little-loop region so that the little-hook region can attach to the little-loop region when the free end of the flexible strap(s) is folded over the core region of the elongated main body of said flexible strap(s), and thus forming a fixation loop.
- at least some of the markings are arranged in the little-loops region of the elongated main body of the strap(s).
- at least one flexible strap comprises an elongated main body comprising a core region terminated by the free end, said core region comprising the little-loop region and the free end comprising little-hook region, preferably at least two straps.
- the markings comprise numbers, letters, symbols, drawings, icons or means of indication, or a combination thereof.
- the outer faces of at least two flexible straps comprise the markings, preferably of at least four flexible straps.
- the outer faces of several flexible straps comprise at least 3 markings, preferably at least 4 markings.
- the outer faces of several flexible straps comprise several markings arranged in a row.
- the markings are juxtaposed and spaced from each other.
- the markings are equidistant from each other.
- the markings are spaced from each other of about between 0,1 cm and 4 cm.
- the markings comprise several increasing numbers (i.e. digits), such as 1, 2, 3, 4... 10....
- the markings comprise several increasing numbers (i.e. digits) associated with small lines or similar forming graduations or the like.
- each marking, especially each number, represents a specific position for positioning the end (e.g. tip) of the flexible straps, thereby helping the patient to correctly set or adjust the mask in choosing/selecting a suitable tightening-up force to be applied to the straps.
- the markings are printed, painted, silk-screened, embossed, engraved, heat transferred or otherwise applied on the outer face of the flexible straps.
- the flexible straps are integral with a connecting portion, i.e. attached to a common central part.
- the connecting portion has an X-shape or the like.
- at least two of said flexible straps are linked, i.e. attached, to one another by means of an arch-forming strap forming an arch that is contact with the top of the head of the patient, when the headgear is worn by said patient.
- the flexible straps and the arch-forming strap, and preferably the connecting portion, form a tridimensional structure dimensioned for matching the contours of the head of the patient, when the headgear is worn by said patient.
- the flexible straps have a length of about between 4 cm and 30 cm.
- the flexible straps have a width of about between 0,5 cm and 4 cm preferably of about between 1 cm and 3 cm.
- the flexible straps material are made of variable elasticity, i.e. can be (slightly) stretchable or not.
- the flexible straps have good fatigue mechanical properties.
- the headgear edges might be open or close.
- the headgear parts are welded or stitched together.

The disclosure also concerns a respiratory mask comprising a mask body, two lateral arms and an upper arm, said two lateral arms and said upper arm comprise free distal ends carrying headgear connecting structures means configured for attaching the flexible straps of a headgear thereto, wherein the free ends of the flexible straps of a headgear according to the present disclosure are attached to said headgear connecting structures means.

The respiratory mask comprising a headgear according to the present disclosure can further comprise one or more of the following additional features:
- the headgear connecting structures means comprise slots, hooks, passage or the like, and combinations thereof.
- the headgear connecting structures means of the respiratory mask, especially slots and/or hooks, cooperate with the fixation loops of the headgear.
- the mask body is made of polymeric material, such as polypropylene (PP), polycarbonate (PC) and nylon, or other similar materials.
- the two lateral arms and the upper arm are arranged on either a front shroud attached to the mask body or on the mask body itself.
- the fixation loops of the headgear are attached to slots and/or hooks carried by the two lateral arms and the upper arm that are attached to the mask body or to the front shroud attached to the mask body.
- the lateral arms projects laterally and the upper arm projects upwardly with respect to the mask body.
- the mask body comprises a gas inlet in fluid communication with an inner chamber, i.e. a respiratory chamber, wherein the patient inhales or exhales gases.
- the front shroud comprising a central aperture.
- a tubular elbow connector (i.e. curved connector) comprising a mask connecting end is plugged into, i.e. snap-fitted into, the gas inlet of the mask body, said elbow connector comprising an inner passage in fluid communication with the inner chamber of the mask body.
- the mask connecting end of the elbow connector pivots into the gas inlet of the mask body, i.e. they form a ball-and-socket mechanism.
- the front shroud is fixed to the elbow connector so that the central aperture of the front shroud is a coaxially arranged with the gas inlet of the mask body and with the mask connecting end of the elbow connector.
- the gas inlet of the mask body comprises a collar element arranged around the gas inlet and projecting outwardly.
- the upwardly-projecting upper arm, the front shroud and/or the two lateral arms are made of at least one plastic material, such as polycarbonate (PC), polypropylene (PP) or nylon.
- the upper arm of the front shroud is slightly flexible for being able to slightly pivot or bend toward the mask body, when a tension force is applied by the headgear.
- the front shroud is mounted in front of the mask body, i.e. surrounding, enveloping or wrapping at least a part of the peripheral outer surface of the mask body.
- the front shroud is held integrally with the mask body by the hollow connector, i.e. the front shroud is sandwiched between the hollow connector and the mask body.
- the respiratory mask is a nasal or facial mask that is dimensioned for covering/receiving either the nose or the nose and the mouth of the patient.
- the elbow connector comprises a tubular body traversed by an inner passage and a tubular sleeve arranged around the tubular body, said tubular sleeve delimiting a venting passage between the tubular sleeve and the tubular body and comprising a plurality of venting holes putting the venting passage in fluid communication with the atmosphere. Preferably, the venting holes are arranged in a circular arc. The venting ports, which are orifices that allow the venting of CO₂-enriched gas to the atmosphere, i.e. gas expired the patient that wears the mask. Preferably, the venting ports have a generally tronconical shape and preferably a size, such as an outlet diameter, of between about 0,3 mm and 3 mm, i.e. measured at their exit on the side of the surrounding atmosphere.
- the elbow connector is designed and adapted for conveying a gas, such as air under pressure (i.e. > 1 bar).
- the respiratory mask further comprises an anti-asphyxia system (AAS) comprising a venting port cooperating with a mobile flap. Such an AAS is known from US-A-5438981 and WO-A-00/38772. Such AAS are useful in facial masks in case of failure of the gas delivery apparatus.
- the mask body is a hollow body, also called mask shell, comprising the inner chamber and the gas inlet orifice in fluid communication with said inner chamber for allowing gas entering into said inner chamber (during inspiration phases) by said inlet orifice.
- the gas inlet orifice has a diameter of about between 1 and 3 cm.
- the gas inlet orifice is arranged substantially centrally in the mask body.
- the mask body has a generally tridimensional (3D) shape, preferably having a general contour that is substantially triangular or trapezoidal.
- the tubular connector comprises a tubular upstream portion for receiving and securing thereto a hose or gas line by means of a connecting piece, such as an intermediary tubular connector that is rotatable with respect to the elbow connector.
- the respiratory mask further comprises a flexible cushion fixed to the mask body, for instance it can be snap-fitted to the mask body, or fixed thereto by any other means, such as glued to it or even molded in one piece with the mask body.
- the flexible cushion comprises a large aperture/opening configured, e.g. dimensioned, for receiving either only the nose of the patient (i.e. in the case of a nasal mask) or both the nose and the mouth of the patient (i.e. in the case of a facial mask), in use, i.e., when the patient wears the mask.
- the respiratory mask is preferably a facial mask covering, in use, the nose and the mouth of the patient. In this embodiment, the cushion is a facial cushion configured and sized for coming into contact, when the mask is worn by the patient, with specific regions of the user's face such as the nasal bridge region and the lateral nose regions, the chin region, i.e. the area located under the lower lip, and the cheek regions located on each lateral sizes of the nose and mouth, said cheek regions linking the nasal bridge region including lateral nose regions to the chin region. The nasal bridge region can be an area of the nose located either at the junction of bone and cartilage, or below or above said bone/cartilage junction of the nose; the chin region is expending from the lower lip to the chin, whereas the cheek regions are the areas of the face located on right and left sizes of the nose and mouth.
- the cushion can comprise one (or several) membrane(s) forming a kind of flexible skirt along its large aperture that deforms so as to match the contours of the face of the patient thereby ensuring a better gas tightness, when the patient wears the mask.
- the cushion is made of a soft flexible material, preferably silicone or similar materials.
- the upwardly-projecting upper arm (or holding arm) faces, i.e. in use, at least a part of the nose and of the forehead of the patient.
- the two lateral arms project, in use, laterally (i.e. on the right and left sides) along part of the cheeks of the patient.
- in an embodiment, the two lateral arms and/or the upwardly-projecting upper arm are integrally fixed to the front shroud, for instance molded in one-piece or glued together.
- in another embodiment, the two lateral arms and/or the upwardly-projecting upper arm are integrally fixed to the mask body, for instance molded in one-piece or glued together.
- the upper arm and/or the two lateral arms have an elongated shape, for example a band or ribbon shape. Of course, other shapes are possible.
- the upper arm has a length of about 2 and 8 cm, or any other suitable shape.
- the lateral arms have a length of about 3 and 6 cm, or any other suitable shape.
- at least a region of the upper arm and/or of the two lateral arms is configured or shaped so as to match, i.e. to match, the external shape, i.e. the outer contours, of the mask body, when the front shroud is fixed to the mask body. Preferably, they have a curved-shape.

The disclosure also concerns an assembly for delivering a gas to a patient comprising a gas delivery device, such as a medical ventilator, and a respiratory mask and headgear according to the present disclosure.

Preferably the gas delivery device is fluidly connected to the respiratory mask by means of a gas line or conduit, such as a flexible hose.

The medical ventilator comprises a motorized turbine or blower that delivers an airflow that is further conveyed by the flexible hose or the like, to the respiratory mask that is arranged on the patient's face and hold in position by the headgear of the invention.

Embodiments of a headgear according to the present disclosure and of a respiratory mask equipped with such a headgear according to the present disclosure are shown in the enclosed illustrative, but not limitative, Figures, among which:
- Figure 1 represents a general view of an embodiment of a respiratory mask for connecting a headgear according to the present disclosure thereto, as shown in Figures 2 and 3.
- Figure 2 is a general view of an embodiment of a headgear according to the present disclosure.
- Figure 3 is an enlarged view of a part of a strap of the headgear of Figure 2.
- Figure 4 is an enlarged view of a part of a strap of the headgear of Figure 2 wherein the user has folded the flexible strap so that the detachable securing means is removably attached to another region of the flexible strap so that the flexible strap makes a large fixation loop around the connecting structures of the mask.
- Figure 5 is a view of a part of a strap similar to Figure 3.
- Figure 6 is a view of a part of a strap, when folded, similar to Figure 4.

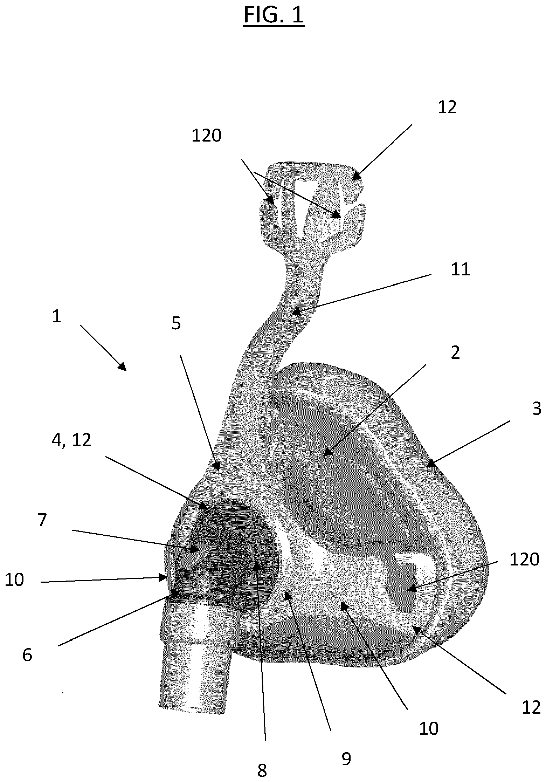

Figure 1 represent general view of an embodiment of a respiratory mask 1, such as a facial or nasal mask, preferably nasal mask, comprising a mask body or shell 2 comprising a gas inlet 4 in fluid communication with an inner chamber (not shown), also called a respiratory chamber.

The mask body 2 further comprises a flexible cushion 3, for instance made of silicone or the like, comprising an enlarged opening (not visible) fluidly communicating with the inner chamber and dimensioned for receiving the nose or nose and mouth of the patient so that said patient can breathe the gas in said inner chamber.

The flexible cushion 3 has a tridimensional hollow structure, such as a triangular, trapezoidal or saddle shape or the like, so as to better fit with the contours of the patient's face, especially in the nasal and/or mouth regions. The flexible cushion 3 is fixed to the rear side of the mask body 2, for instance by a snap-fit connection, a "sandwich"-type connection or the like, or they can also be glued together or the like.

A hollow curved connector 6, i.e. an elbow-shape tubular connector, is fluidly connected to the gas inlet 4 for providing the respiratory gas to the inner chamber, such as air or an air/oxygen mixture that can be inhaled by the patient during inspiratory phases.

The hollow connector 6 is fed with gas, such as pressurized air or an air/O₂ mixture, by a flexible hose (not shown), or the like, conveying pressurized gas delivered by a gas source, such as a medical ventilator or apparatus.

The hollow curved connector 6 comprises a safety valve 7 forming at least a part of an anti-asphyxia system allowing the patient breathing fresh air through the safety valve 7 in case of failure of the gas source, such as a medical ventilator, and further comprises venting orifices 8 for venting the CO₂-enriched gas expired by the patient during expiratory phases. Anti-asphyxia systems are known in the art, as disclosed by US-A-5438981 or WO-A-00/38772.

A front shroud 5, also called "retainer" or "front piece", is arranged on the mask body 2. The shroud 5 comprises a central aperture 12 that is traversed by the hollow connector 6. The central aperture 12 of the front shroud 5 and the gas inlet 4 of the mask body 2 are coaxially arranged.

The front shroud 5 comprises two lateral arms 10 and an upper arm 11 that are integrally fixed to or molded in one-piece with the rest of the front shroud 5, i.e. the shroud body 9.

When the front shroud 5 is fixed to the mask body 2, the upper arm 11 projects upwardly, i.e. about vertically, with respect to the mask body 2, whereas the two lateral arms 10 project laterally from said mask body 2 in opposite directions, i.e. one toward the right side of the mask 1 and the other toward the left side of the mask 1.

The upper arm 11 and/or of the two lateral arms 10 have elongated shapes, for example band or ribbon shapes, and are made of a material, such as a polymer material, e.g. polycarbonate (PC), polypropylene (PP) or nylon so as to be slightly bendable toward the patient's face under the forces applied by the headgear, when said headgear is adjusted to the morphology of the patient's head. The front shroud 5 can be made of the same polymer material or of any other suitable material.

In another embodiment (not shown), the upper arm 11 and/or of the two lateral arms 10 can be directly arranged on the mask body 2, i.e. held by the mask body 2, without using any front shroud 5.

Generally speaking, the mask body 2 can be made from a unique piece or from several pieces or sub-units that are subsequently assembled together and made integral by means of any available fixing technique, for instance they can be glued, thermo-welded or similarly affixed together. It can be made of a polymer or plastic material, such as polypropylene (PP), polycarbonate (PC) and nylon, or the like.

When the facial mask 1 is worn by the patient, i.e. when the enlarged opening of cushion 3 receives the nose or the nose and the mouth of the patient, while the patient breathes the gas contained in the inner compartment or exhales CO₂-enriched gases into it, the cushion 3 is in contact with particular and well defined regions of the patient's face, thereby ensuring gas tightness.

In some embodiments, the cushion 3 can comprise one or several membranes forming a peripheral skirt participating to or improving gas tightness.

For instance, the cushion 3 can be a facial cushion configured and sized for coming into contact, when the mask is worn by a user, with at least the nasal bridge region (including lateral nose regions), the chin region, i.e. the area located under the lower lip, and the cheek regions located on both lateral sizes of the nose and mouth of the patient, said cheek regions linking the nasal bridge region including lateral nose regions to the chin region. The nasal bridge region can be an area of the nose located either at the junction of bone and cartilage, or below or above said bone/cartilage junction of the nose; the chin region is expanding from the lower lip to the chin, whereas the cheek regions are the opposite areas located on right and left sizes of the nose and mouth. Of course, other embodiments and shapes of the cushion 2 are possible.

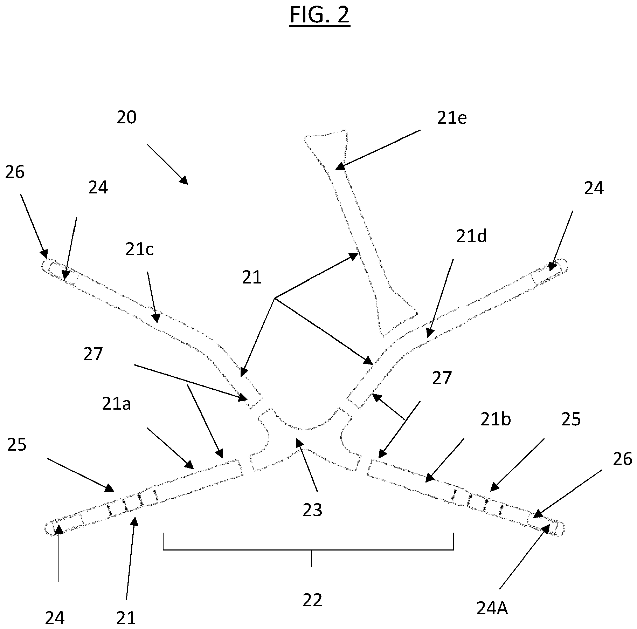

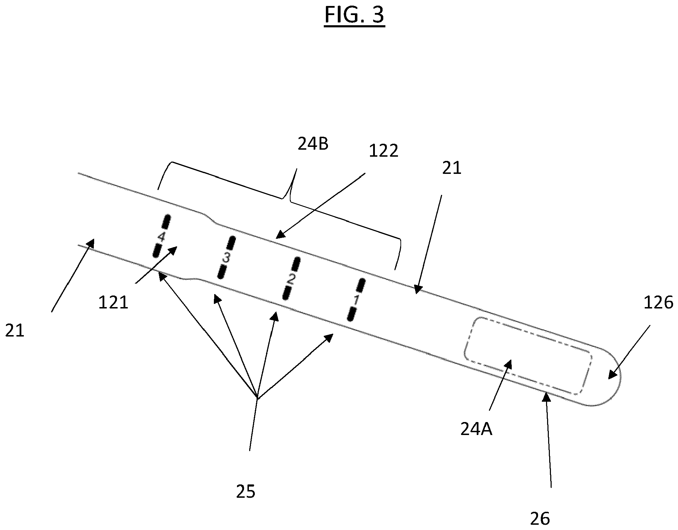

As already mentioned, depending on the embodiment, the two lateral arms 10 and the upper arm 11 can be attached either to the front shroud 5 as shown in Figure 1 or directly to the mask body 2. They are configured for receiving and holding a flexible headgear 20 that is used for securing and maintaining the mask 1 in position on the patient's face, when the mask is used, i.e. worn by the patient. To this end, the two lateral arms 10 and the upper arm 11 comprise free distal ends carrying headgear connecting structures 12 or means, such as slots, hooks, passages or the like, so as to receive the straps 21 of the headgear 20 of Fig. 2 and 3.

As shown in Figures 2 and 3, the headgear 20 according to the present disclosure comprises several flexible straps 21a-21d, forming part of a flexible tridimensional structure 22 that matches the shape, i.e. morphology, of the patient's head, when worn by the patient.

As shown in the Figures, the straps 21a-21d are preferably elongated ribbons, bands, strips or the like. Further, the straps 21a-21d are flexible and can be folded. They can be slightly resilient, i.e. stretchable, or not.

In the embodiment of Figure 2, the tridimensional structure 22 forming the headgear 20 according to the present disclosure comprises a connecting portion 23 having, in this embodiment, an "X" shape and four straps 21a-21d secured, i.e. attached, thereto, i.e. to the four "arms" of the X-shape, by their securing ends 27. Of course, the connecting portion 23 can have other shapes.

A fifth strap or arch-forming strap 21e has its two opposite ends attached to straps 21c, 21d, forming a bridge or arch in-between that is in contact with the top of the head of the patient, when the headgear 20 is worn by said patient.

Generally speaking, the flexible straps 21a-21d each comprise an elongated main body 21 terminated by a free end 26.

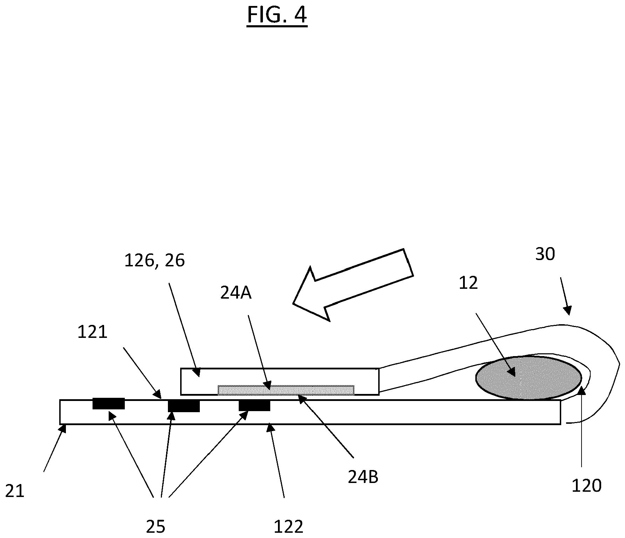

In the embodiment shown in Figures 3-6, the flexible straps 21a-21d comprise detachable securing-means 24A, 24B for removable attachment, for instance a loop/hook connection means, such as a Velcro^{®}-type system. For attaching the flexible straps 21a-21d to the mask 1, the user has for example first to pass the free end 26 of each of said flexible straps 21a-21d into a lodging 120 (or hook) of the connecting structures 12 of the two lateral arms 10 and the upper arm 11, such as slot, hook or the like. Then, as shown in Fig. 4, the user has to fold, i.e. to bend, the flexible strap 21a-21d so that the detachable securing-means 24 can removably attach. Once folded, each flexible strap 21a-21d makes a large fixation loop 30 around the connecting structures 12 of the mask 1, namely slots, hooks or the like.

Preferably, each of the detachable securing means 24A, 24B comprises Velcro^{®}-type regions, as shown in Fig. 2-6, including a little-loops region 24B comprising a multitude of little loops and a little-hooks region 24A comprising a multitude of little hooks, that cooperate together for ensuring the removable attachment.

Preferably, the little-hooks region 24A is arranged close to the free end 26 of the strap(s) 21a-21d, whereas the little-loops region 24B is arranged in the main body 21 of the strap(s) 21a-21d, namely in the core region 122 of the straps 21a-21d, thereby allowing the free end 26 of the strap(s) 21a-21d to be folded over the main body 21 of the strap(s) 21a-21d, thereby forming the fixation loop 30, and further attached thereto thanks to a cooperation of the little-hooks region 24A with the little-loops region 24B of the detachable securing means 24A, 24B.

At least a part of the flexible straps 21a-21d, i.e. at least the little-loops region 24B, can be made of fabric material or the like exhibiting the multitude of little loops. Each little-loop region 24B is arranged on the outer face 121 of the elongated main body 21 (i.e. in the core region 122) of the flexible strap(s) 21a-21d, i.e. on the face of the strap that is oriented toward the outside and not in contact with the head of the patient, when the headgear 20 is worn by the patient, as visible on Fig. 3 and 4. Fabric material can comprise natural or synthetic material, such as polymer fibers or the like. Preferably, (at least) the elongated main body 21 of each flexible straps 21a-21d is (almost) entirely made of said fabric material so that each little-loops region 24B can adhere to the multitude of little-hooks regions 24A arranged on the outer face 121 of each flexible strap 21a-21d, typically in the region of the free ends 26.

Similarly, each little-hooks region 24A is carried by, i.e. arranged on, the outer face 121 of the flexible strap(s) 21a-21d, i.e. in the region of their free ends 26. The little hooks of the little-hooks region 24A can be made of plastic material, i.e. polymer or the like.

In other words, thanks to the detachable securing means 24A, 24B forming a hook and loop fastener, the free end 26 of each flexible strap 21a-21d can be folded over and attached to the outer face 121 of the core region 122 of the main body 21 of the flexible straps 21a-21d so as to adopt different positions along the main body 21 of the flexible straps 21a-21d, that are represented by the markings 25 and designated by the tips 126 forming the extremity of the free end 26 of the flexible straps 21a-21d, as below explained, when the straps 21a-21d of the headgear are tightened up by the user.

This allows the user to easily modify (i.e. set or re-set) the tightening up (i.e. traction or pulling forces) applied to the mask 1 for better adjusting the headgear 20 is to the morphology of the user, or also to retrieve a previous setting.

Indeed, as already explained, when the mask is positioned on the face of a user, such as a patient, so that the mask fits the facial morphology of said user, and maintained in said desired position, this tightening up action may lead to an excessive crushing of the cushion on the nasal bridge region, when the straps of the headgear of the mask are overly tightened. This may lead to gas leaks and discomfort for the patient. Obviously, a loose tightening can also lead to similar issues, i.e. gas leaks and discomfort for the patient. In both cases, the mask is less efficient for delivering a gas therapy, which is not acceptable.

According to the present disclosure, for solving such a problem, the headgear 20 of the disclosure comprises several markings 25 arranged, in the embodiment shown in the Figures, on two of the four flexible straps 21a-21d, namely on the two lower straps 21a-21b. Said markings 25 are carried by the outer face 121 of the straps 21a-21b, i.e. arranged in their core regions 122, so as to be visible by the user from the outside, for instance by reflection in a mirror.

The markings 25 correspond to different tightening-up settings (i.e. 'stretching position') of the flexible straps 21a-21b. In other words, each marking 25 represents a given position that can be selected among the possible different positions that can adopt the free end 26, especially the end tip 126, of the flexible straps 21a-21b when they are folded by the user, thereby forming a fixation loop 30 that can be mechanically connected/attached to the mask 1, namely to the slots, hooks or the like.

The markings 25 can be numbers, letters, symbols, icons, drawings or the like. Markings 25 can be printed, painted, silk-screened, embossed, engraved or heat transferred on the outer face 121 of the flexible straps 21a-21d. Preferably, markings 25 comprise numbers 250 (i.e. digits), e.g. 1, 2, 3... 10, associated with little lines 251 forming graduations as shown in Figures 3, 5 and 6.

The markings 25 can be arranged on all the flexible straps 21a-21d or only of part of them, for instance on two flexible straps 21a-21d as shown in the Figures, such as the two lower straps 21a, 21b that are attached to both lateral sides (i.e. right and left sides) of the mask 1 (i.e. mask body or front shroud) of Figure 1.

Preferably, the markings 25 (or some of them) are arranged in (all or part of) the little-loops region 24B of the elongated main body 21 of the strap(s) 21a-21b.

Generally speaking, when a flexible strap 21a-21b comprising markings 25 is folded by the user, its free end 26 comprising the end tip 126 comes over the outer face 121 of the core region 122 of its elongated main body 21 and is thus positioned vis-à-vis one of the several markings 25, such as a 'digit/graduation' association, corresponding to a given or known tightening-up (i.e. tensional force), so as to provide an indication to the user of the corresponding tightening-up setting.

Thanks to the cooperation between the markings 25 (i.e. digits 250/graduations 251) and the tips 126 located at the extremity of the free end 26 of the strap(s) 21a-21d, the user can easily find the most appropriated setting, i.e. positioning, of each the ends 26/tips 126 of the flexible straps 21a-21d and hence better control the tightening up applied by each flexible strap 21a-21d on the mask 1.

In other words, markings 25 constitute locators or the like and the tips 126 constitute indicators or the like that assist the user in well-positioning the headgear 20 and the mask 1, especially in setting a suitable/efficient tightness of the straps.

Once the right markings 15 are known, the user will be also able to quickly put again his/her mask 1 in its optimal position, without wasting time and avoiding false positioning, for instance every evening when the user puts it on before falling asleep.

If the mask is nevertheless not well positioned, for instance due to loose straps (or conversely, to an excessive tightening-up), the user can easily remove the loose straps, by pulling on their tips 126 that are facing "wrong" markings 25, and then reposition the straps towards other markings 25, namely "right marketings/settings", so that their tips 126 are repositioned vis-à-vis different markings 25, i.e. in more suitable locations, thereby obtaining a better tightening up of the mask 1 on the patient's face.

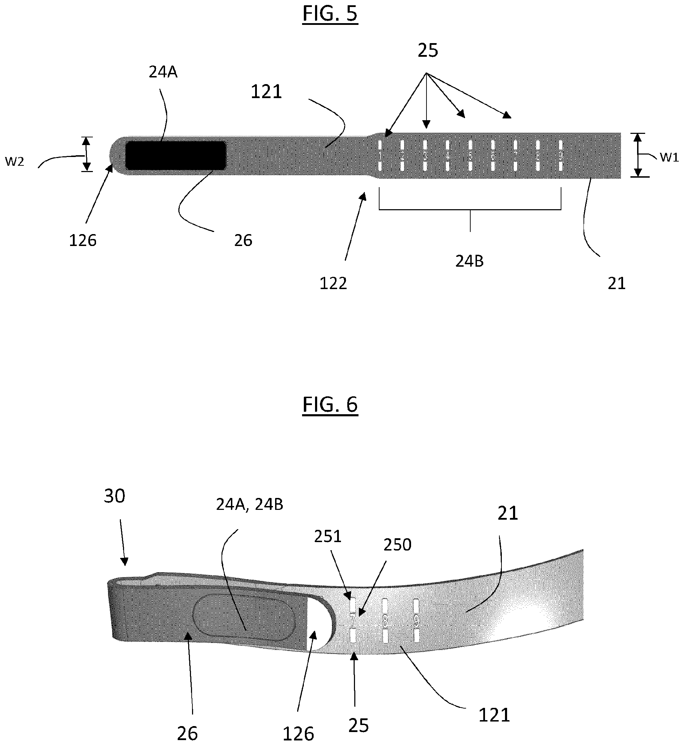

As shown in Figure 5, the elongated main body 21 comprises a core region 122 having a first width W1 and a free end 26 having a second width W2, wherein the first width W1 is greater than the second width W2, i.e. W1 > W2. For instance, W1 can be comprised between 6 and 30 mm, whereas W2 can be comprised between 4 and 25 mm. Having different widths W1, W2 facilitates the positioning of the free end 26 with respect to the markings arranged in the core region 122 of the strap(s).

Generally speaking, the respiratory mask 1 of the present invention, such as a facial or nasal mask, is light, comfortable to wear, easy to position and to secure on the patient's face, and provide a good tightness, i.e. gas seal. It can be used in a method for treatment of a respiratory disorder or condition affecting infant, toddler and child patients as well as adult patients.

## Claims

1. Respiratory mask (1) comprising a headgear (20) comprising several flexible straps (21a-21d), wherein at least one of said flexible straps (21a-21d) :
- comprises an elongated main body (21) comprising a core region (122) terminated by a free end (26) with a tip (126), an outer face (121) located externally, and detachable securing means (24A, 24B) arranged on said outer face (121),
- is configured for being foldable so that, when folded, the free end (26) of said flexible strap (21a-21d) can be positioned by a user in several different positions located along the outer face (121) of said at least one flexible strap (21a-21d) and removably secured in one of said several different positions by means of the detachable securing means (24A, 24B),
- and further comprises several markings (25) arranged on the outer face (121) of said at least one flexible strap (21a-21d), said several markings (25) corresponding to different tightenings of said at least one flexible strap (21a-21d),
wherein :
- the markings (25) comprise several increasing numbers (250), and
- when said at least one flexible strap (21a-21d) of the headgear (20) is folded, the tip (126) of the free end (26) of said at least one flexible strap (21a-21d) is positioned vis-à-vis one given marking of said several markings (25) so as to designate said given marking thereby assisting the user in choosing a desired tightening of said at least one flexible strap (21a-21d),
**characterized in that** :
- the detachable securing means (24A, 24B) of the headgear (20) comprise a little-loop region (24B) comprising a multitude of little loops and a little-hook region (24A) comprising a multitude of little hooks cooperating together for ensuring a desired removable attachment of the little-hook region (24A) to the little-loop region (24B),
- the core region (122) of said at least one flexible strap (21a-21d) of the the headgear (20) comprises the little-loop region (24B) and the free end (26) comprises the little-hook region (24A), and
- the markings are arranged in the little-loops region (24B) of the elongated main body (21) of at least one flexible strap (21a-21d) of the the headgear (20),
and wherein :
- the little-hook region (24A) arranged on the elongated main body (21) is configured for being attached to the little-loop region (24B) arranged on the free end (26) of said at least one flexible strap (21a-21d), when said free end (26) is folded over the core region (122) of said elongated main body (21), thereby forming a fixation loop (30), and
- when folded, the free end (26) of one or several flexible straps (21a-21d) of the headgear (20) is maintained along and in contact with the outer face (121) of said at least one flexible strap (21a-21d) by means of the detachable securing means (24A, 24B), in a desired position wherein the tip (126) of the free end (26) of said one or several flexible straps (21a-21d) designates said one given marking of the several markings (25).

2. Respiratory mask according to Claim 1, **characterized in that** the headgear (20) comprises at least two flexible straps (21a-21d) comprising detachable securing means (24A, 24B) and markings (25).

3. Respiratory mask according to any one of Claims 1 or 2, **characterized in that** the flexible straps (21a-21d) of the headgear (20) are made of a flexible material chosen among fabric and polymer materials.

4. Respiratory mask according to Claim 1, **characterized in that** the tip (126) of the free end (26) of said at least one flexible strap (21a-21d) of the headgear (20) is made of polymer material.

5. Respiratory mask according to Claim 1, **characterized in that** the markings (25) comprise several increasing numbers (250) associated with small lines forming graduations (251).

6. Respiratory mask according to Claim 1, **characterized in that** at least two (21c, 21d) of said flexible straps (21a-21d) of the headgear (20) are linked to one another by an arch-forming strap (21e) forming an arch that is contact with the top of the head of the patient, when the headgear (20) is worn by said patient.

7. Respiratory mask (1) according to Claim 1, **characterized in that** it further comprises a mask body (2), two lateral arms (10) and an upper arm (11), said two lateral arms (10) and said upper arm (11) comprise free distal ends carrying headgear connecting structures means (12), the free ends (26) of the flexible straps (21a-21d) of the headgear (20) being attached to said headgear connecting structures means (12).

8. Respiratory mask (1) according to Claim 7, **characterized in that** the headgear connecting structures means (12) comprise slots, hooks or passages.

9. Respiratory mask (1) according to Claim 7, **characterized in that** said two lateral arms (10) and said upper arm (11) are arranged on a front shroud (5) attached to the mask body (2), said lateral arms (10) projecting laterally and said upper arm (11) projecting upwardly.

10. Respiratory mask according to claims 1 to 9, **characterized in that** it is a facial or nasal mask, preferably a nasal mask (1).

11. Respiratory mask according to claim 1, **characterized in that** the tip (126) having :
- a flat rounded-shape, such as a flat half-disk shape, or
- a flat angular-shape, such as a triangular or a trapezoidal flat shape.

12. Respiratory mask according to claim 1, **characterized in that** at least two flexible straps comprise the free end (26) with a colorized tip (126) comprising a colored layer or structure.

13. Respiratory mask according to claim 12 4 , **characterized in that** the colored layer or structure is at least partially made of polymer material.

14. Assembly for delivering a gas to a patient comprising a gas delivery device, preferably a medical ventilator, and the respiratory mask (1) with a headgear according to any one of claims 1 to 13.

## Patentansprüche

1. Atemmaske (1), umfassend eine Kopfmontur (20), die mehrere flexible Bänder (21a - 21d) umfasst, wobei eines der flexiblen Bänder (21a - 21d):
- einen länglichen Hauptkörper (21) umfasst, der einen Kernbereich (122), der von einem freien Ende (26) mit einer Spitze (126) begrenzt wird, eine außen angeordnete Außenfläche (121) und ablösbare Befestigungsmittel (24A, 24B), die an der Außenfläche (121) angeordnet sind, umfasst,
- faltbar ausgestaltet ist, so dass das freie Ende des flexiblen Bands (21a - 21d), wenn Letzteres gefaltet ist, von einem Benutzer in mehreren unterschiedlichen Positionen, die entlang der Außenfläche (121) des mindestens einen flexiblen Bands (21a - 21d) angeordnet sind, positioniert und mittels der ablösbaren Befestigungsmittel (24A, 24B) in einer der mehreren unterschiedlichen Positionen entfernbar befestigt werden kann,
- und ferner mehrere Markierungen (25) umfasst, die an der Außenfläche (121) des mindestens einen flexiblen Bands (21a - 21d) angeordnet sind, wobei die mehreren Markierungen (25) unterschiedlichen Anspannungen des mindestens einen flexiblen Bands (21a - 21d) entsprechen,
wobei:
- die Markierungen (25) mehrere aufsteigende Zahlen (250) umfassen und
- die Spitze (126) des freien Endes (26) des mindestens einen flexiblen Bands (21a - 21d), wenn das mindestens eine flexible Band (21a - 21d) der Kopfmontur (20) gefaltet ist, gegenüber einer gegebenen Markierung der mehreren Markierungen (25) positioniert ist, um die gegebene Markierung zu bezeichnen, wodurch dem Benutzer dabei geholfen wird, eine gewünschte Anspannung des mindestens einen flexiblen Bands (21a - 21d) zu wählen, **dadurch gekennzeichnet, dass**:
- die ablösbaren Befestigungsmittel (24A, 24B) der Kopfmontur (20) einen Bereich (24B) mit kleinen Schlaufen, der eine Vielzahl von kleinen Schlaufen umfasst, und einen Bereich (24A) mit kleinen Haken umfassen, der eine Vielzahl von kleinen Haken umfasst, die zur Sicherstellung einer gewünschten entfernbaren Anbringung des Bereichs (24A) mit kleinen Haken an dem Bereich (24B) mit kleinen Schlaufen zusammenwirken,
- der Kernbereich (122) des mindestens einen flexiblen Bands (21a - 21d) der Kopfmontur (20) den Bereich (24B) mit kleinen Schlaufen umfasst und das freie Ende (26) den Bereich (24A) mit kleinen Haken umfasst und
- die Markierungen in dem Bereich (24) mit kleinen Schlaufen des länglichen Hauptkörpers (21) des mindestens einen flexiblen Bands (21a - 21d) der Kopfmontur (20) angeordnet sind,
und wobei:
- der an dem länglichen Hauptkörper (21) angeordnete Bereich (24A) mit kleinen Haken dazu ausgestaltet ist, an dem Bereich (24B) mit kleinen Schlaufen, der an dem freien Ende (26) des mindestens einen flexiblen Bands (21a - 21d) angeordnet ist, angebracht zu werden, wenn das freie Ende (26) über den Kernbereich (122) des länglichen Hauptkörpers (21) gefaltet wird, wodurch eine Befestigungsschlaufe (30) gebildet wird, und
- das freie Ende (26) eines oder mehrerer flexibler Bänder (21a - 21d) der Kopfmontur (20), wenn es gefaltet ist, mittels der ablösbaren Befestigungsmittel (24A, 24B) in einer gewünschten Position entlang und in Kontakt mit der Außenfläche (121) des mindestens einen flexiblen Bands (21a - 21d) gehalten wird, wobei die Spitze (126) des freien Endes (26) des einen oder der mehreren flexiblen Bänder (21a - 21d) die eine gegebene Markierung der mehreren Markierungen (25) bezeichnet.

2. Atemmaske nach Anspruch 1, **dadurch gekennzeichnet, dass** die Kopfmontur (20) mindestens zwei flexible Bänder (21a - 21d) umfasst, die ablösbare Befestigungsmittel (24A, 24B) und Markierungen (25) umfassen.

3. Atemmaske nach einem der Ansprüche 1 oder 2, **dadurch gekennzeichnet, dass** die flexiblen Bänder (21a - 21d) der Kopfmontur (20) aus einem flexiblen Material hergestellt sind, das aus Gewebe und Polymermaterialien ausgewählt ist.

4. Atemmaske nach Anspruch 1, **dadurch gekennzeichnet, dass** die Spitze (126) des freien Endes (26) des mindestens einen flexiblen Bands (21a - 21d) der Kopfmontur (20) aus Polymermaterial hergestellt ist.

5. Atemmaske nach Anspruch 1, **dadurch gekennzeichnet, dass** die Markierungen (25) mehrere aufsteigende Nummern (250) umfassen, die kleinen Linien zugeordnet sind, die Teilstriche bilden.

6. Atemmaske nach Anspruch 1, **dadurch gekennzeichnet, dass** mindestens zwei (21c, 21d) der flexiblen Bänder (21a - 21d) der Kopfmontur (20) über ein bogenbildendes Band (21e) miteinander verbunden sind, das einen Bogen bildet, der mit der Oberseite des Kopfs des Patienten in Kontakt ist, wenn die Kopfmontur (20) von dem Patienten getragen wird.

7. Atemmaske (1) nach Anspruch 1, **dadurch gekennzeichnet, dass** sie ferner einen Maskenkörper (2), zwei seitliche Arme (10) und einen oberen Arm (11) umfasst, wobei die beiden seitlichen Arme (10) und der obere Arm (11) freie distale Enden umfassen, die Kopfmonturverbindungsstrukturmittel (12) tragen, wobei die freien Enden (26) der flexiblen Bänder (21a - 21d) der Kopfmontur (20) an den Kopfmonturverbindungsstrukturmitteln (12) angebracht sind.

8. Atemmaske (1) nach Anspruch 7, **dadurch gekennzeichnet, dass** die Kopfmonturverbindungsstrukturmittel (12) Schlitze, Haken oder Durchgänge umfassen.

9. Atemmaske (1) nach Anspruch 7, **dadurch gekennzeichnet, dass** die beiden seitlichen Arme (10) und der obere Arm (11) an einer an dem Maskenkörper (2) angebrachten vorderen Abdeckung (5) angeordnet sind, wobei die seitlichen Arme (10) seitlich vorragen und der obere Arm (11) nach oben vorragt.

10. Atemmaske nach Ansprüchen 1 bis 9, **dadurch gekennzeichnet, dass** sie eine Gesichts oder eine Nasenmaske und vorzugsweise eine Nasenmaske (1) ist.

11. Atemmaske nach Anspruch 1, **dadurch gekennzeichnet, dass** die Spitze (126) Folgendes aufweist:
- eine flache abgerundete Form wie eine flache Halbscheibenform oder
- eine flache winklige Form wie eine dreieckige oder trapezförmige flache Form.

12. Atemmaske nach Anspruch 1, **dadurch gekennzeichnet, dass** mindestens zwei flexible Bänder das freie Ende (26) mit einer farbigen Spitze (126) umfassen, die eine farbige Schicht oder Struktur umfasst.

13. Atemmaske nach Anspruch 12, **dadurch gekennzeichnet, dass** die farbige Schicht oder Struktur mindestens teilweise aus Polymermaterial hergestellt ist.

14. Baugruppe zum Zuführen eines Gases zu einem Patienten, umfassend eine Gaszuführungsvorrichtung, vorzugsweise ein medizinisches Beatmungsgerät, und eine Atemmaske (1) mit einer Kopfmontur nach einem der Ansprüche 1 bis 13.

## Revendications

1. Masque respiratoire (1) comprenant un harnais (20) comprenant plusieurs sangles souples (21a à 21d), au moins l'une desdites sangles souples (21a à 21d) :
- comprenant un corps principal allongé (21) comprenant une région de coeur (122) terminée par une extrémité libre (26) dotée d'une pointe (126), une face externe (121) située à l'extérieur, et des moyens de fixation détachables (24A, 24B) placés sur ladite face externe (121),
- étant conçue pour pouvoir être pliée de sorte que, lorsqu'elle est pliée, l'extrémité libre (26) de ladite sangle souple (21a à 21d) peut être positionnée par un utilisateur dans plusieurs positions différentes situées le long de la face externe (121) de ladite au moins une sangle souple (21a à 21d) et fixée de manière amovible dans l'une desdites plusieurs positions différentes à l'aide des moyens de fixation détachables (24A, 24B),
- et comprenant en outre plusieurs marquages (25) placés sur la face externe (121) de ladite au moins une sangle souple (21a à 21d), lesdits plusieurs marquages (25) correspondant à des serrages différents de ladite au moins une sangle souple (21a à 21d),
dans lequel :
- les marquages (25) comprennent plusieurs nombres croissants (250), et
- lorsque ladite au moins une sangle souple (21a à 21d) du harnais (20) est pliée, la pointe (126) de l'extrémité libre (26) de ladite au moins une sangle souple (21a à 21d) est positionnée vis-à-vis d'un marquage donné desdits plusieurs marquages (25) afin d'indiquer ledit marquage donné, aidant ainsi l'utilisateur à choisir un serrage souhaité de ladite au moins une sangle souple (21a à 21d),
**caractérisé en ce que** :
- les moyens de fixation détachables (24A, 24B) du harnais (20) comprennent une région de petites boucles (24B) comprenant une multitude de petites boucles et une région de petits crochets (24A) comprenant une multitude de petits crochets coopérant ensemble pour assurer une fixation amovible souhaitée de la région de petits crochets (24A) à la région de petites boucles (24B),
- la région de coeur (122) de ladite au moins une sangle souple (21a à 21d) du harnais (20) comprend la région de petites boucles (24B) et l'extrémité libre (26) comprend la région de petits crochets (24A), et
- les marquages sont placés dans la région de petites boucles (24B) du corps principal allongé (21) d'au moins une sangle souple (21a à 21d) du harnais (20),
et dans lequel :
- la région de petits crochets (24A) placée sur le corps principal allongé (21) est conçue pour être fixée à la région de petites boucles (24B) placée sur l'extrémité libre (26) de ladite au moins une sangle souple (21a à 21d), lorsque ladite extrémité libre (26) est repliée sur la région de coeur (122) dudit corps principal allongé (21), formant ainsi une boucle de fixation (30), et
- lorsqu'elle est pliée, l'extrémité libre (26) d'une ou de plusieurs sangles souples (21a à 21d) du harnais (20) est maintenue le long de la face externe (121), et en contact avec celle-ci, de ladite au moins une sangle souple (21a à 21d) à l'aide des moyens de fixation détachables (24A, 24B), dans une position souhaitée la pointe (126) de l'extrémité libre (26) de ladite ou desdites plusieurs sangles souples (21a à 21d) indiquant ledit marquage donné des plusieurs marquages (25).

2. Masque respiratoire selon la revendication 1, **caractérisé en ce que** le harnais (20) comprend au moins deux sangles souples (21a à 21d) comprenant des moyens de fixation détachables (24A, 24B) et des marquages (25).

3. Masque respiratoire selon l'une quelconque des revendications 1 ou 2, **caractérisé en ce que** les sangles souples (21a à 21d) du harnais (20) sont fabriquées en un matériau souple choisi parmi des matériaux en tissu et polymères.

4. Masque respiratoire selon la revendication 1, **caractérisé en ce que** la pointe (126) de l'extrémité libre (26) de ladite au moins une sangle souple (21a à 21d) du harnais (20) est fabriquée en un matériau polymère.

5. Masque respiratoire selon la revendication 1, **caractérisé en ce que** les marquages (25) comprennent plusieurs nombres croissants (250) associés à de petites lignes formant des graduations (251).

6. Masque respiratoire selon la revendication 1, **caractérisé en ce qu'**au moins deux (21c, 21d) desdites sangles souples (21a à 21d) du harnais (20) sont reliées l'une à l'autre par une sangle formant une arche (21e) formant une arche qui est en contact avec le sommet de la tête du patient, lorsque le harnais (20) est porté par ledit patient.

7. Masque respiratoire (1) selon la revendication 1, **caractérisé en ce qu'**il comprend en outre un corps de masque (2), deux bras latéraux (10) et un bras supérieur (11), lesdits deux bras latéraux (10) et ledit bras supérieur (11) comprennent des extrémités distales libres supportant des moyens formant structures de raccordement de harnais (12), les extrémités libres (26) des sangles souples (21a à 21d) du harnais (20) étant fixées auxdits moyens formant structures de raccordement de harnais (12) .

8. Masque respiratoire (1) selon la revendication 7, **caractérisé en ce que** les moyens formant structures de raccordement de harnais (12) comprennent des fentes, des crochets ou des passages.

9. Masque respiratoire (1) selon la revendication 7, **caractérisé en ce que** lesdits deux bras latéraux (10) et ledit bras supérieur (11) sont placés sur une coiffe frontale (5) fixée au corps de masque (2), lesdits bras latéraux (10) faisant saillie latéralement et ledit bras supérieur (11) faisant saillie vers le haut.

10. Masque respiratoire selon les revendications 1 à 9, **caractérisé en ce qu'**il s'agit d'un masque facial ou nasal, préférablement d'un masque nasal (1).

11. Masque respiratoire selon la revendication 1, **caractérisé en ce que** la pointe (126) présente :
- une forme arrondie plate, telle qu'une forme de demi-disque plate, ou
- une forme angulaire plate, telle qu'une forme plate triangulaire ou trapézoïdale.

12. Masque respiratoire selon la revendication 1, **caractérisé en ce qu'**au moins deux sangles souples comprennent l'extrémité libre (26) dotée d'une pointe (126) colorée comprenant une couche ou structure colorée.

13. Masque respiratoire selon la revendication 12, **caractérisé en ce que** la couche ou structure colorée est au moins partiellement fabriquée en matériau polymère.

14. Ensemble pour administrer un gaz à un patient comprenant un dispositif d'administration de gaz, de préférence un ventilateur médical, et le masque respiratoire (1) doté d'un harnais selon l'une quelconque des revendications 1 à 13.
